(19) Europäisches Patentamt
European Patent Office
Office européen des brevets

(11) Veröffentlichungsnummer: **0 200 686**
**A2**

# (12) EUROPÄISCHE PATENTANMELDUNG

(21) Anmeldenummer: 86810191.6

(22) Anmeldetag: 28.04.86

(51) Int. Cl.[4]: **C 07 C 131/00**
C 07 C 155/02, C 07 C 149/32
C 07 D 317/54, A 01 N 39/00
A 01 N 47/24

(30) Priorität: 02.05.85 CH 1868/85
04.06.85 CH 2364/85
21.03.86 CH 1144/86

(43) Veröffentlichungstag der Anmeldung:
05.11.86 Patentblatt 86/45

(84) Benannte Vertragsstaaten:
BE CH DE FR GB IT LI NL

(71) Anmelder: CIBA-GEIGY AG
Klybeckstrasse 141
CH-4002 Basel(CH)

(72) Erfinder: Karrer, Friedrich, Dr.
Rebbergstrasse 5
CH-4800 Zofingen(CH)

(54) **Oximderivate.**

(57) Neue Oximderivate der Formel

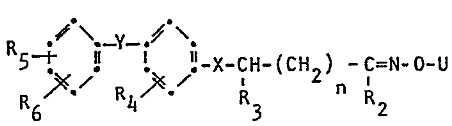

worin
U für einen der Reste

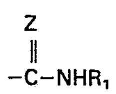 , 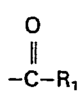 oder $R_1$

steht und wobei
$R_1$ $C_1$-$C_6$-Alkyl, $C_2$-$C_5$-Alkenyl, $C_3$-Monohalogenalkenyl, $C_3$-$C_5$-Alkinyl, $C_3$-$C_6$-Cycloalkyl, $C_2$-$C_5$-Alkoxyalkyl, $C_2$-$C_5$-Alkylthioalkyl oder gegebenenfalls am Kern substituiertes Phenyl oder Benzyl,
$R_2$ und $R_3$ unabhängig voneinander Wasserstoff oder $C_1$-$C_3$-Alkyl,
$R_4$, $R_5$ und $R_6$ unabhängig voneinander Wasserstoff, Halogen, $C_1$-$C_4$-Alkyl, $C_2$-$C_4$-Halogenalkyl, $C_1$-$C_4$-Alkoxy, $C_1$-$C_4$-Halogenalkoxy oder Nitroc,
n eine Zahl 0 oder 1,

X Sauerstoff, Schwefel oder $CH_2$,

Y Sauerstoff, Schwefel, $CH_2$ oder C=O und

Z Sauerstoff oder Schwefel bedeuten; mit der Massgabe, dass X und Y nicht gleichzeitig Sauerstoff bedeuten und/oder mindestens einer der Reste $R_4$, $R_5$ und $R_6$ eine von Wasserstoff abweichende Bedeutung hat, wenn $R_1$ eine $C_1$-$C_3$-Alkylgruppe darstellt;
Verfahren zur Herstellung dieser Oximderivate und ihre Verwendung in der Schädlingsbekämpfung insbesondere als Ovizide gegen Insekten und Vertretern der Ordnung Akarina.

EP 0 200 686 A2

86810197.6 0200686

CIBA-GEIGY AG

Basel (Schweiz)

5-15333/1-3/+

## Oximderivate

Die vorliegende Erfindung betrifft Oximderivate, Verfahren zu ihrer Herstellung und ihre Verwendung in der Schädlingsbekämpfung.

Die erfindungsgemässen Oximderivate (Oximcarbamate, Oximester und Oximäther) haben die Formel

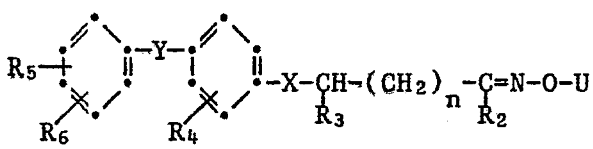 (I),

worin

U für einen der Reste

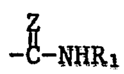 , 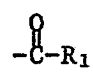 oder $R_1$

steht und wobei

$R_1$ $C_1$-$C_6$-Alkyl, $C_2$-$C_5$-Alkenyl, $C_3$-Monohalogenalkenyl, $C_3$-$C_5$-Alkinyl, $C_3$-$C_6$-Cycloalkyl, $C_2$-$C_5$-Alkoxyalkyl, $C_2$-$C_5$-Alkylthioalkyl, oder gegebenenfalls am Kern substituiertes Benzyl,

$R_2$ und $R_3$ unabhängig voneinander Wasserstoff oder $C_1$-$C_3$-Alkyl,

$R_4$, $R_5$ und $R_6$ unabhängig voneinander Wasserstoff, Halogen, $C_1$-$C_4$-Alkyl, $C_2$-$C_4$-Halogenalkyl, $C_1$-$C_4$-Alkoxy, $C_1$-$C_4$-Halogenalkoxy oder Nitro,

n eine Zahl 0 oder 1,

X Sauerstoff, Schwefel oder $>CH_2$,

Y Sauerstoff, Schwefel, $>CH_2$ oder $>C=O$ und

Z Sauerstoff oder Schwefel bedeuten;

mit der Massgabe, dass X und Y nicht gleichzeitig Sauerstoff bedeuten und/oder mindestens einer der Reste $R_4$, $R_5$ und $R_6$ eine von Wasserstoff abweichende Bedeutung hat, wenn $R_1$ eine $C_1$-$C_3$-Alkylgruppe darstellt.

Halogen steht im Rahmen der vorliegenden Erfindung für Fluor, Chlor, Brom oder Jod, vorzugsweise für Fluor oder Chlor.

Der Erfindungsgegenstand umfasst auch die möglichen Isomeren der Verbindungen der Formel I.

Die Alkyl-, Alkoxy-, Halogenalkyl-, Halogenalkoxy-, Alkoxyalkyl, Alkylthioalkyl-, Alkenyl- und Alkinylgruppen können geradkettig oder verzweigt sein. Beispiele solcher Gruppen sind u.a. Methyl, Methoxy, Methoxymethyl, Methylthiomethyl, Difluormethoxy, Aethyl, Aethoxy, 2-Fluoräthoxy, 1,1,2,2-Tetrafluoräthyl, Pentafluoräthyl, 1,1,2,2-Tetrafluoräthoxy, Pentafluoräthoxy, Propyl, Isopropyl, n-Butyl, n-Pentyl, n-Hexyl und deren Isomere, Vinyl, 1-Propen-3yl, 1-Propinyl.

Bevorzugte Cycloalkylgruppen bei $R_1$ sind Cyclopentyl und Cyclohexyl.

Als Substituenten an den Phenyl- oder Benzylgruppen bei $R_1$ kommen bevorzugt Halogen, $C_1$-$C_4$-Alkyl, $C_1$-$C_4$-Alkoxy, $C_1$-$C_4$-Alkylthio oder $C_1$-$C_4$-Halogenalkyl in Frage.

Bevorzugt sind erfindungsgemäss Verbindungen der Formel I,
worin
$R_1$ $C_1$-$C_6$-Alkyl, $C_2$-$C_5$-Alkenyl, $C_3$-Chloralkenyl, $C_3$-$C_5$-Alkinyl oder gegebenenfalls am Kern substituiertes Benzyl;
$R_2$ und $R_3$ unabhängig voneinander Wasserstoff oder Methyl;
$R_4$, $R_5$ und $R_6$ Wasserstoff,
n eine Zahl 0 oder 1;

X Sauerstoff, Schwefel oder $>CH_2$;
Y Sauerstoff oder $>CH_2$ und

Z Sauerstoff oder Schwefel bedeuten.

Insbesondere bevorzugt sind Verbindungen der Formel I, die dadurch gekennzeichnet sind, dass
U für einen der Reste

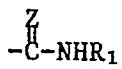 , 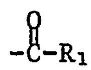 , $-CH_2-CH=CHCl$ ,

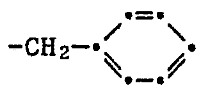 , 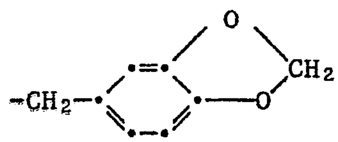 , $-CH_2-C\equiv CH$

oder $-CH_2-CH=CH_2$ steht
und $R_1$, $R_2$, $R_3$, $R_4$, $R_5$, $R_6$, n, X, Y und Z die vorstehend angegebenen Bedeutungen haben, mit der Massgabe, dass Y und X gleichzeitig Sauerstoff, sowie $R_2$ Methyl bedeuten und/oder, dass mindestens einer der Reste $R_4$, $R_5$ oder $R_6$ eine von Wasserstoff abweichende Bedeutung hat, wenn U für den Rest $-CH_2-CH=CH_2$ steht.

Hervorzuheben sind wegen ihrer biologischen Bedeutung auch die erfindungsgemässen Verbindungen der Formel Ia

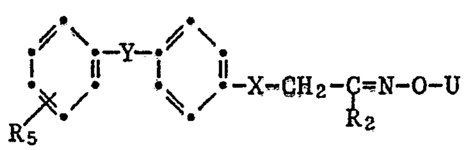 (Ia),

die dadurch gekennzeichnet sind, dass
U für einen der Reste

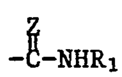 , $-CH_2-CH=CHCl$ , 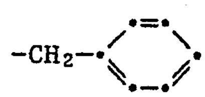 ,

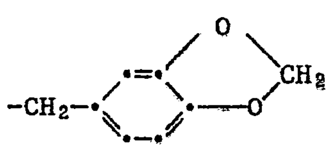 oder $-CH_2-CH=CH_2$

steht und

$R_1$ $C_1$-$C_4$-Alkyl;

$R_2$ Wasserstoff oder Methyl;

$R_5$ Wasserstoff, Halogen, Methyl oder Trifluormethyl;

X und Z unabhängig voneinander Sauerstoff oder Schwefel; und

Y Sauerstoff oder $>CH_2$ bedeuten, mit der Massgabe, dass X und Y gleichzeitig Sauerstoff und $R_2$ Methyl bedeuten und/oder, dass $R_3$ eine von Wasserstof abweichende Bedeutung hat, wenn U für den Rest $-CH_2-CH=CH_2$ steht.

Die Verbindungen der Formel I bzw. Ia) können nach an sich bekannten Methoden wie folgt hergestellt werden:

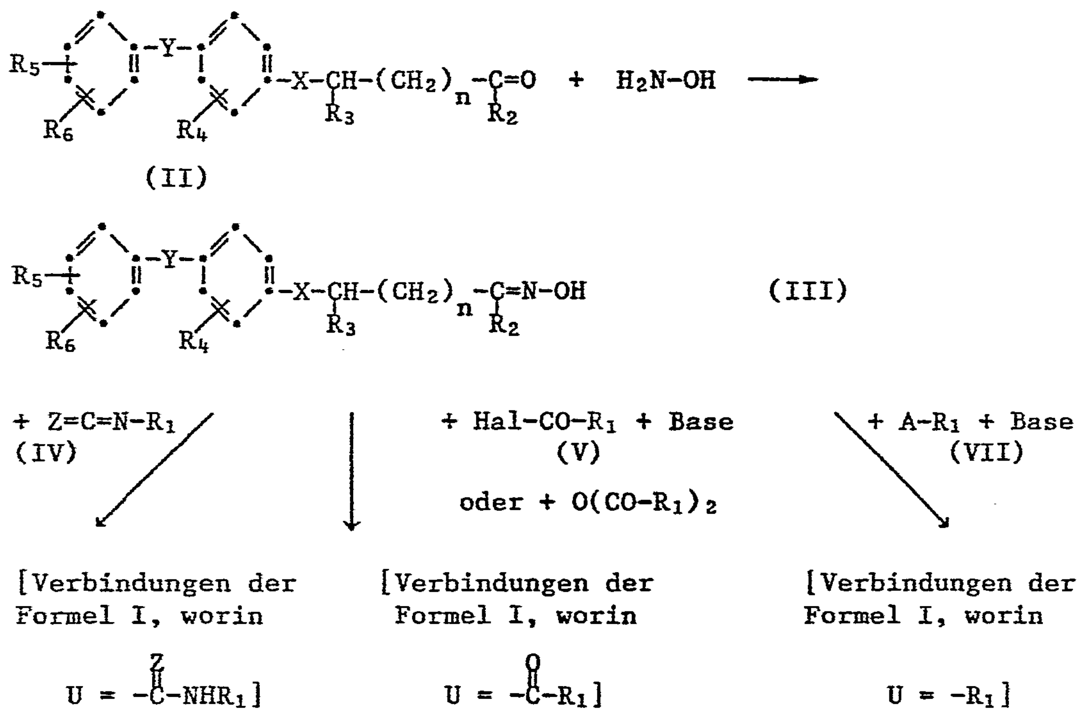


In den Formeln II bis VI haben $R_1$ bis $R_6$, n, X, Y und Z die vorstehend für Formel I bzw. Ia) angegebene Bedeutungen. In der Formel V steht Hal für ein Halogenatom, vorzugsweise Chlor. In der Formel VII steht A für eine herkömmliche Abgangsgruppe, wie z.B. ein Halogenatom, vorzugsweise Chlor, die Mesyloxy- oder Tosyloxy-Gruppe. Als Basen kommen insbesondere tertiäre Amine, wie Trialkylamine und Pyridin, ferner Hydride, Hydroxide, Oxide, Carbonate und Hydrogencarbonate von Alkali- und Erdalkalimetallen sowie Alkalimetallalkoholate, wie z.B. Kalium-t-butylat und Natriummethylat usw. in Betracht.

Bei der vorstehend beschriebenen Herstellung der erfindungsgemässen Verbindungen wird also aus einer Carbonylverbindung der Formel II mit Hydroxylamin zunächst das Oxim der Formel III gebildet, das dann a) mit einem Isocyanat oder Isothiocyanat der Formel IV oder b) mit einem Acylhalogenid der Formel V in Gegenwart einer Base oder mit einem Anhydrid der Formel VI oder c) in Gegenwart einer Base mit einer Verbindung der Formel VII umgesetzt wird.

Die Verfahrensschritte werden bei einer Reaktionstemperatur zwischen -10 und +150°C, zumeist zwischen +20 und +80°C, bei normalem oder erhöhtem Druck und vorzugsweise in einem inerten Lösungs- oder Verdünnungsmittel durchgeführt. Als Lösungs- oder Verdünnungsmittel eignen sich z.B. Aether und ätherartige Verbindungen, wie Diäthyläther, Diisopropyläther, Dioxan, 1,2-Dimethoxyäthan und Tetrahydrofuran; Amide, wie N,N-dialkylierte Carbonsäureamide; aliphatische, aromatische sowie halogenierte Kohlenwasserstoffe, insbesondere Benzol, Toluol, Xylole, Chloroform und Chlorbenzol; Nitrile wie Acetonitril; Dimethylsulfoxid und Ketone wie Aceton und Methyläthylketon.

Zur Herstellung von Verbindungen der Formel I bzw. Ia, worin U den Rest $R_1$ bedeutet, kann man auch eine Carbonyl-Verbindung der Formel II, worin $R_2$ bis $R_6$ n, X und Y die vorstehend angegebenen Bedeutungen haben, direkt mit einem Hydroxylaminhydrohalogenid-Derivat der Formel VIII

$$\left[ \overset{\oplus}{H_3N}\text{-O-}R_1 \right] \quad Hal^{\ominus} \qquad \text{(VIII)}$$

in Gegenwart einer Base, z.B. eines Alkaliacetats, umsetzten, wobei in Formel VIII der Rest $R_1$ die vorstehend angegebenen Bedeutungen hat und Hal für ein Halogenatom, z.B. Chlor, steht. Für dieses Verfahren werden Alkohole oder Alkohol-Wasser-Gemische als Lösungsmittel bevorzugt.

Die Ausgangsstoffe der Formeln II bis VIII sind bekannt oder können, falls sie neu sind, analog bekannten Methoden hergestellt werden. Sie bilden dann ebenfalls einen Gegenstand der vorliegenden Erfindung.

Die Verbindungen der Formel I können als Gemische von verschiedenen geometrischen resp. enantiomeren Formen vorliegen, bzw. bei der Synthese gebildet werden, wenn bei der Herstellung nicht einheitliche Ausgangsmaterialien verwendet werden. Die Isomerengemische können nach bekannten Methoden in die einzelnen Formen aufgetrennt werden. Unter der Verbindung der Formel I versteht man sowohl die einzelnen geometrischen resp. enantiomeren Formen, als auch deren Gemische.

Die Verbindungen der Formel I eignen sich zur Bekämpfung von verschiedenartigen Schädlingen an Tieren und Pflanzen sowie im Boden. So können sie zur Bekämpfung von Insekten, z.B. der Ordnung Lepidoptera, Coleoptera, Homoptera, Heteroptera, Diptera, Thysanoptera, Orthoptera, Anoplura, Siphonaptera, Mallophaga, Thysanura, Isoptera, Psocoptera und Hymenoptera und von Milben und Zecken der Ordnung Acarina eingesetzt werden.

So eignen sich Verbindungen der Formel I bzw. Ia) zur Bekämpfung von pflanzenschädigenden Insekten in Zier- und Nutzpflanzen, insbesondere in Baumwollkulturen (z.B. Spodoptera littoralis und Heliothis virescens). Die Verbindungen der Formel I haben auch eine günstige Wirkung gegen Bodeninsekten (z.B. Aulacophora femoralis, Chortophila brassicae, Diabrotica balteata, Pachnoda savignyi und Scotia ypsilon). Insbesondere aktiv sind die erfindungsgemässen Verbindungen als Ovizide im Pflanzenschutz, speziell zur Bekämpfung von pflanzenschädigenden Insekten, wie z.B. Laspeyresia pomonella und Lobesia botrana.

Wirkstoffe der Formel I zeigen auch eine sehr günstige Wirkung gegen Fliegen, wie z.B. Musca domestica, sowie Mückenlarven. Allgemein zeichnen sie sich durch eine breite ovizide und ovolarvizide Wirkung

aus. Sie besitzen gute Wirkung gegen ektoparasitäre Milben und Zecken z.B. der Familien Ixodidae, Argasidae und Dermanyssidae und können die Eiablage ektoparasitärer Akariden hemmen.

Die akarizide bzw. insektizide Wirkung lässt sich durch Zusatz von anderen Insektiziden und/oder Akariziden wesentlich verbreitern und an gegebene Umstände anpassen. Als Zusätze eignen sich z.B. org. Phosphorverbindungen; Nitrophenole und deren Derivate; Formamidine; Harnstoffe; pyrethrinartige Verbindungen sowie Karbamate und chlorierte Kohlenwasserstoffe.

Die gute pestizide Wirkung der erfindungsgemässen Verbindungen der Formeln I und Ia entspricht einer Abtötungsrate (Mortalität) von mindestens 50-60 % der erwähnten Schädlinge.

Mit besonderem Vorteil werden Verbindungen der Formel I auch mit Substanzen kombiniert, welche einen synergistischen oder verstärkenden Effekt ausüben. Beispiele solcher Verbindungen sind u.a. Piperonylbutoxid, Propinyläther, Propinyloxime, Propinylcarbamate und Propinylphosphonate, 2-(3,4-Methylendioxyphenoxy)-3,6,9-trioxaundecan (Sesamex resp. Sesoxane), S,S,S-Tributylphosphorotrithioate, 1,2-Methylendioxy-4-(2-(octylsulfinyl)-propyl)-benzol.

Die Verbindungen der Formel I werden in unveränderter Form oder vorzugsweise zusammen mit den in der Formulierungstechnik üblichen Hilfsmitteln eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet. Die Anwendungsverfahren wie Versprühen, Vernebeln, Verstäuben, Verstreuen oder Giessen werden gleich wie die Art der Mittel den angestrebten Zielen und den gegebenen Verhältnissen entsprechend gewählt.

Die Formulierungen, d.h. die mindestens einen Wirkstoff der Formel I und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Aether und Ester, wie Aethanol, Aethylenglykol, Aethylenglykolmonomethyl- oder -äthyläther, Essigester, Propylmyristat oder Propylpalmitat, Ketone wie Cyclohexanon, stark polare Lösungsmittel wie N-Methyl-2-pyrrolidon, Dimethylsulfoxid oder Dimethylformamid, sowie gegebenenfalls epoxidierte Pflanzenöle wie epoxidiertes Kokosnussöl oder Sojaöl; Silikonöle oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen, wie z.B. Bimsstein, Ziegelbruch, Sepiolit oder Bentonit, als nicht sorptive Trägermaterialien z.B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes der Formel I nichtionogene, kation- und/oder anionaktive Tenside mit guten Emulgier-, Dispergier-und Netzeigenschaften in Betracht. Unter Tensiden sind auch Tensidgemische zu verstehen.

Geeignete anionische Tenside können sowohl sog. wasserlösliche Seifen wie wasserlösliche synthetische oberflächenaktive Verbindungen sein.

Als Seifen eignen sich die Alkali-, Erdalkali- oder gegebenenfalls substituierten Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Tallöl gewonnen werden können. Ferner sind auch die Fettsäuremethyl-taurinsalze zu erwähnen.

Häufiger werden jedoch sog. synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2 Sulfonsäuregruppen und einen Fettsäurerest mit 8-22 C-Atomen. Alkylarylsulfonate sind z.B. die Na-, Ca- oder Triäthanolaminsalze der Dodecylbenzolsulfonsäure, der Dibutylnaphthalinsulfonsäure, oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Aethylenoxid-Adduktes und Phospholipide in Frage.

Als nichtionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Aethylenglykoläthergruppen und 10 bis 100 Propylenglykoläthergruppen enthaltenden Polyäthylenoxid-Addukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykoleinheit 1 bis 5-Aethylenglykoleinheiten.

Als Beispiele nichtionischer Tenside seien Nonylphenolpolyäthoxyäthanole, Ricinusölpolyglycoläther, Ricinusölthioxilat, Polypropylen-Polyäthylenoxid-Addukte, Tributylphenoxypolyäthoxyäthanol, Polyäthylenglykol und Octylphenoxypolyäthoxyäthanol erwähnt.

Ferner kommen auch Fettsäureester von Polyoxyäthylensorbitan wie das Polyoxyäthylensorbitan-trioleat in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Aethylsulfate vor, z.B. das Stearyltrimethylammoniumchlorid oder das Benzyldi(2-chloräthyl)-äthylammoniumbromid.

Die in der Formulierungstechnik gebräuchlichen Tenside sind u.a. in folgenden Publikationen beschrieben:

"McCutcheon's Detergents and Emulsifiers Annual" MC Publishing Corp., Ridgewood, New Jersey, 1979; Dr. Helmut Stache "Tensid Taschenbuch", Carl Hanser Verlag München/Wien 1981.

Die pestiziden Zubereitungen enthalten in der Regel 0,1 bis 99 %, insbesondere 0,1 bis 95 %, Wirkstoff der Formel I, 1 bis 99,9 % eines festen oder flüssigen Zusatzstoffes und 0 bis 25 %, insbesondere 0,1 bis 25 %, eines Tensides.

Während als Handelsware eher konzentrierte Mittel bevorzugt werden, verwendet der Endverbraucher in der Regel verdünnte Mittel.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Beispiel 1:

a) Herstellung von 4-Phenoxy-phenoxy-acetaldehyd-diäthylacetal:
Eine Lösung von 112 g (0,6 Mol) 4-Phenoxy-phenol und 157,6 g Bromacetaldehyd-diäthylacetal (0,8 Mol) in 480 ml Dimethylformamid wird mit 150 g Kaliumcarbonat und 4 g feinpulverigem Kaliumjodid versetzt und unter Stickstoff 14 Std. auf 150°C erhitzt. Nun werden aus der Reaktionslösung die Salze abfiltriert und im Wasserstrahlvakuum das Dimethylformamid weitgehend abdestilliert. Der Rückstand wird in Aether gelöst, zweimal mit Wasser, dreimal mit 20%iger Kalilauge und wieder mit Wasser gewaschen, die Aetherlösung über Natriumsulfat getrocknet und der Aether vollständig abdestilliert.

Das so erhaltene 4-Phenoxy-phenoxy-acetaldehyd-diäthylacetal wird ohne weitere Reinigung zur Herstellung des freien Aldehyds verwendet.

b) Herstellung von 4-Phenoxy-phenoxy-acetaldehyd:
Die Lösung von 170 g des unter a) erhaltenen Acetals in 2000 ml Tetrahydrofuran wird mit 280 ml 1N-HCl versetzt und unter Stickstoff 21 Std. bei 45°C gerührt. Anschliessend wird das Tetrahydrofuran im Vakuum abdestilliert, der Rückstand mit Diäthyläther versetzt und wiederholt mit gesättigter Natriumhydrogencarbonat-Lösung und schliesslich mit Wasser gewaschen. Die Aetherphase trocknet man über Natriumsulfat und destilliert das Lösungsmittel vollständig ab. Der rohe Aldehyd wird an Kieselgel chromatographisch gereinigt, (Eluierungsmittel: Diäthyläther-Hexan 1:1) wodurch reiner 4-Phenoxy-phenoxy-acetaldehyd vom Smp. 79-81°C erhalten wird.

c) Herstellung von 4-Phenoxy-phenoxy-acetaldehydoxim:
Zu einer Lösung von 68,5 g 4-Phenoxy-phenoxy-acetaldehyd in 300 ml Aethanol wird bei Raumtemperatur die Lösung von 25 g Hydroxylamin-hydrochlorid und 29,4 g kristallwasserfreiem Natriumacetat in 100 ml Wasser getropft. Nach dem Abflauen der leicht exothermen Reaktion wird das Gemisch 2 Std. auf Rückflusstemperatur erwärmt. Nun wird auf 0°C abgekühlt und das Gemisch 3 Std. bei dieser Temperatur gerührt, wobei das gebildete Oxim auskristallisiert. Das auskristallisierte 4-Phenoxy-phenoxy-acetaldehydoxim (Nr. 1) abgenutscht, wiederholt gut mit Wasser gewaschen und im Vakuumschrank bei 40°C getrocknet. Smp. 102-105°C.

d) Herstellung von 4-Phenoxy-phenoxy-acetaldehydoxim-propargyläther:
Zu einer Suspension von 2,24 g Natriumhydrid (55%ige Suspension in Mineralöl, die vorgängig mit Hexan gewaschen wird) in 50 ml Dimethylformamid (Stickstoff-Atmosphäre) tropft man unter Rühren bei 0-5°C innerhalb von ca. 30 Min. die Lösung von 12,2 g 4-Phenoxy-phenoxy-acetaldehydoxim in 15 ml Dimethylformamid. Nach weiteren 4 Std. Rühren bei 8-10°C ist die Wasserstoffentwicklung beendet. Nun tropft man in ca. 20 Min. bei 0-5°C 8,2 g Propargylbromid zum Reaktionsgemisch und rührt anschliessend über Nacht bei Raumtemperatur. Zur Aufarbeitung wird das Reaktionsgemisch mit Wasser versetzt und wiederholt mit Diäthyläther extrahiert. Die vereinigten Aetherphasen werden mit Wasser gewaschen, über Natriumsulfat getrocknet und das Lösungsmittel abdestilliert. Durch anschliessende chromatographische Reinigung an 250 g Kieselgel (Eluierungsmittel Diäthyläther/n-Hexan 1:9) wird die Titelverbindung der Formel

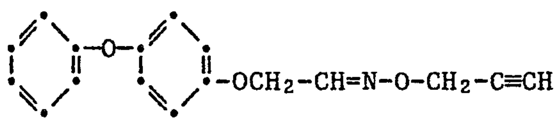

mit einem Brechungsindex von $n_D^{20°} = 1,5710$ (Verbindung Nr. 1) erhalten.

Beispiel 2:

a) Herstellung von 4-Phenoxy-phenoxy-acetonoxim:
Zu einer Lösung von 64 g 4-Phenoxy-phenoxy-aceton in 260 ml Aethanol wird bei Raumtemperatur die Lösung von 22 g Hydroxylamin-hydrochlorid und 26 g wasserfreiem Natriumacetat in 110 ml Wasser in ca. 1 Std. getropft. Nach dem Abflauen der leicht exothermen Reaktion wird noch 3,5 Std. auf Rückflusstemperatur erhitzt. Anschliessend wird aus dem Reaktionsgemisch das Aethanol am Rotationsverdampfer abdestilliert, der wässrige Rückstand dreimal mit Aether-Hexan (2:1) extrahiert, die vereinigten organischen Phasen mit Wasser und 10%iger Natriumhydrogencarbonat-Lösung gewaschen und über Natriumsulfat getrocknet. Nach dem Abdestillieren der Lösungsmittel und der Umkristallisation aus Diäthyläther-Pentan erhält man 4-Phenoxy-phenoxy-acetonoxim vom Smp. 59-61°C.

b) Herstellung von 4-Phenoxy-phenoxy-acetonoxim-äthyläther:
Die Lösung von 7,1 g Acetonoxim-äthyläther (0,07 Mol), 12,1 g 4-Phenoxy-phenoxy-aceton (0,05 Mol) und 100 ml 2N Schwefelsäure in 12 ml 1,2-Dimethoxyäthan wird unter Rühren 12 Std. im schwachen Stickstoff-Strom auf 80°C erwärmt, wobei das abgespaltene Aceton abdestilliert. Hierauf wird die Reaktionslösung mit 100 ml Aether

verdünnt, dreimal mit 10%iger Natriumhydrogencarbonat-Lösung und zweimal mit Wasser gewaschen. Nach dem Trocknen über Natriumsulfat wird das Lösungsmittel abdestilliert und das Rohprodukt an Kieselgel chromatographiert (Eluierungsmittel: Hexan/Aether 19:1), wodurch 4-Phenoxy-phenoxy-acetonoxim-äthyläther (Verbindung Nr. 2) mit dem Brechungsindex $n_D^{20°} = 1,5513$ und der Formel

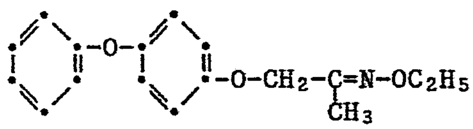

erhalten wird.

Auf analoge Weise werden auch folgende Verbindungen der Formel I hergestellt:

Verbindung Nr. 3 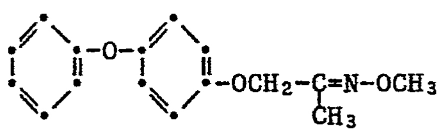 $[n_D^{20°} = 1,5596]$

Verbindung Nr. 4 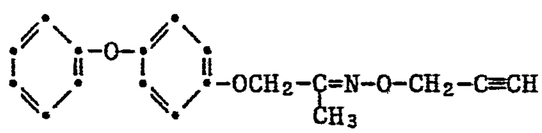 $[n_D^{20°} = 1,5670]$

Beispiel 3:

Herstellung von 4-Phenoxy-phenoxy-acetonoxim-N-methylcarbamat:

Zu einer Lösung von 12,85 g (0,05 Mol) 4-Phenoxy-phenoxy-acetonoxim und 0,05 g Diazabicyclooctan in 90 ml Acetonitril tropft man bei Raumtemperatur unter Rühren in 30 Min. 3,42 g Methylisocyanat (0,06 Mol) in 10 ml Acetonitril. Nach 7 Std. Rühren bei 40°C wird das Lösungsmittel im Rotationsverdampfer abdestilliert und der Rückstand an 200 g Kieselgel chromatographisch gereinigt (Eluierungsmittel: Diäthyläther-Hexan 3:1). Die Hauptfraktion wird

anschliessend in Diäthyläther umkristallisiert, wodurch 4-Phenoxy-phenoxy-acetonoxim-N-methylcarbamat (Verbindung Nr. 5) vom Smp. 60-62°C der Formel

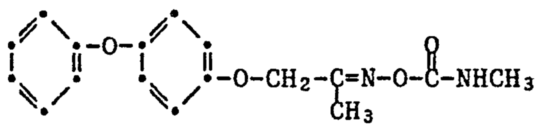

erhalten wird.

Auf analoge Weise wird auch die Verbindung Nr. 6 der Formel

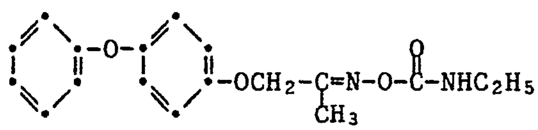

mit einem Schmelzpunkt von 57-59°C erhalten.

Beispiel 4:

Herstellung von 4-Phenoxy-phenoxy-acetonoxim-propionsäureester:
Zu einer Lösung von 10,3 g (0,04 Mol) 4-Phenoxy-phenoxy-acetonoxim und 0,145 g (0,0012 Mol) 4-Dimethylaminopyridin in 30 ml Toluol tropft man unter Rühren bei 60°C innerhalb von 10 Min. 5,9 g (0,045 Mol) Propionsäureanhydrid und rührt weitere 2,5 Std. bei 60°C. Hierauf wird das abgekühlte Reaktionsgemisch mit 50 ml Aether verdünnt, dreimal mit eiskalter 20%iger Natriumcarbonat-Lösung gewaschen, die organische Phase über Natriumsulfat getrocknet und die Lösungsmittel abdestilliert. Der Rückstand wird an 200 g Kieselgel chromatographiert (Eluierungsmittel: Diäthyläther-n-Hexan 1:2) und anschliessend in Diäthyläther-Pentan (1:3) umkristallisiert, wodurch reiner 4-Phenoxy-phenoxy-acetonoxim-propionsäureester der Formel

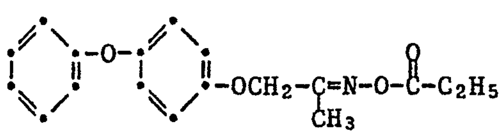

vom Smp. 62-63°C (Verbindung Nr. 7) erhalten wird.

Beispiel 5:

a) Herstellung von 4-(4-Phenoxy-phenyl)-2-butanonoxim:
Zu einer Lösung von 48 g (0,2 Mol) 4-(4-Phenoxy-phenyl)-2-butanon*) in 500 ml Aethanol tropft man bei Raumtemperatur innert ca. 30 Min. die frisch bereitete Lösung aus 15,3 g Hydroxylamin-hydrochlorid (0,22 Mol) in 100 ml Wasser und 200 ml 1N Natronlauge. Anschliessend wird 1,5 Std. auf Rückflusstemperatur erhitzt und 16 Std. bei Raumtemperatur nachgerührt. Das Aethanol wird nun im Wasserstrahlvakuum abdestilliert und der Rückstand wiederholt mit Aether extrahiert. Die vereinigten Aetherphasen wäscht man wiederholt mit Wasser, trocknet über Natriumsulfat und destilliert den Aether vollständig ab. Das rohe Oxim wird an Kieselgel chromatographiert (Eluierungsmittel: Diäthyläther n-Hexan 1:1). Das so erhaltene 4-(4-Phenoxy-phenyl)-2-butanonoxim schmilzt nach Umkristallisation in Diäthyläther n-Hexan bei 80-82°C.
*) z.B. Helv. Chim. Acta 58, 286 (1975)

b) Herstellung von 4-(4-Phenoxy-phenyl)-2-butanonoxim-propargyläther:
2,57 g Natriumhydrid (55%ig in Mineralöl) werden wiederholt mit n-Hexan gewaschen und anschliessend unter Stickstoff in 50 ml Tetrahydrofuran suspendiert. Zu dieser Suspension tropft man in 30 Min. bei Raumtemperatur unter Rühren die Lösung von 15 g 4-(Phenoxyphenyl)-2-butanonoxim in 80 ml Tetrahydrofuran. Anschliessend wird bis zum Ende der Wasserstoffentwicklung bei 50°C weiter gerührt. Nun tropft man bei 20°C die Lösung vond 10,5 g Propargylbromid in 30 ml Hexamethylphosphorsäuretriamid zu und rührt weitere 16 Std. bei Raumtemperatur. Zur Aufarbeitung wird im Rotationsverdampfer erst das Tetrahydrofuran weitgehend abdestilliert, nun fügt man 400 ml Wasser zum Reaktionsgemisch und extrahiert wiederholt mit Aether. Die vereinigten Aetherphasen werden mit Wasser gewaschen, über Natriumsulfat getrocknet und der Aether abdestilliert. Durch

Chromatographie an Kieselgel (Eluierungsmittel:Diäthyläther-n-Hexan/1:3) wird der 4-(4-Phenoxy-phenyl)-2-butanonoxim-propargyläther der Formel

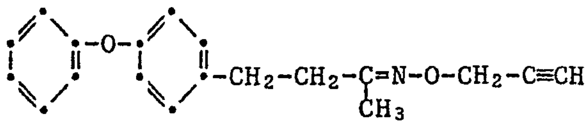

mit einem Brechungsindex von $n_D^{20°} = 1,5647$ (Verbindung Nr. 8) erhalten.

Auf analoge Weise wird auch die Verbindung Nr. 9 der Formel

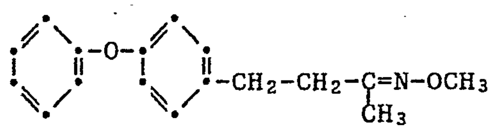

$\left[n_D^{20°} = 1,5597\right]$

hergestellt.

Beispiel 6:

Herstellung von 4-Phenoxy-phenoxy-acetonoxim-allyläther

Zu einer Lösung von 13,3 g (0,055 Mol) 4-Phenoxy-phenoxy-aceton in 70 ml Aethanol tropft man unter Rühren bei 35°C innerhalb von 30 Minuten die Lösung von 7,3 g (0,066 Mol) O-Allylhydroxylamin-hydrochlorid und 5,4 g Natriumacetat (0,066 Mol) in 40 ml Wasser. Nach weiterem 4-stündigem Rühren bei 35°C wird das Aethanol am Rotationsverdampfer abdestilliert und der Rückstand wiederholt mit Diäthyläther extrahiert. Die vereinigten Aetherextrakte werden mit 5%iger Natriumcarbonatlösung und Wasser gewaschen, die organische Phase wird über Natriumsulfat getrocknet, das Lösungsmittel abdestilliert und das Rohprodukt an Kieselgel chromatographisch gereinigt (Eluierungsmittel: n-Hexan/Diäthylether/19:1).

Auf diese Weise erhält man die Titelverbindung der Formel

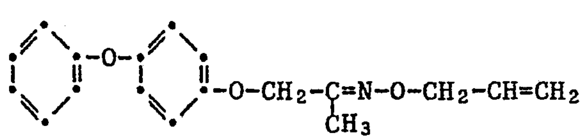

mit einem Brechungsindex $n_D^{23}$ = 1,5572 (Verbindung Nr. 10).

In entsprechender Weise wird auch die Verbindung der Formel

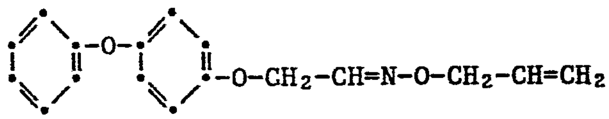

mit dem Brechungsindex $n_D^{18}$ = 1,5633 (Verbindung Nr. 11) hergestellt.

In analoger Weise, wie in den Beispielen 1 bis 6 beschrieben werden auch die folgenden Verbindungen der Formel I hergestellt.

| Verbindung Nr. | | physik. Daten |
|---|---|---|
| 12 | 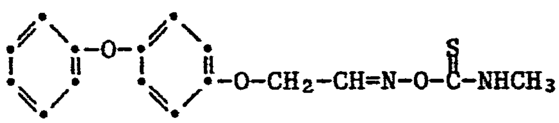 | $n_D^{20}$ = 1,5776 |
| 13 | 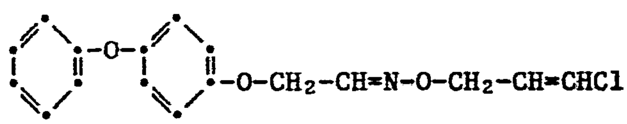 | anti-trans: $n_D^{20}$ = 1,5700<br>syn/cis: $n_D^{20}$ = 1,5720 |
| 14 | 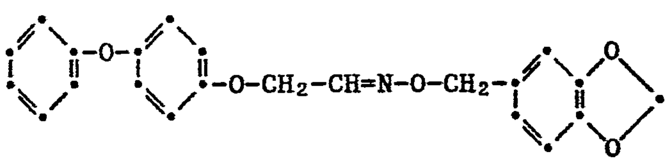 | $n_D^{18}$ = 1,5941 |
| 15 | 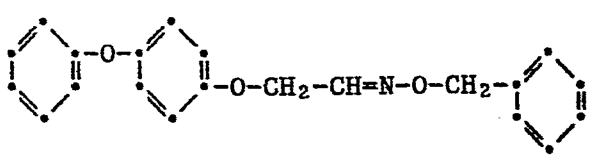 | syn: $n_D^{19}$ = 1,5891<br>anti: Smp. = 56-58°C |

16 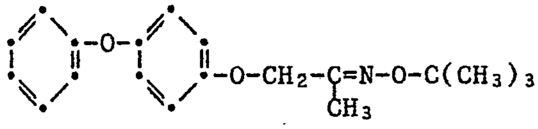 $n_D^{33} = 1,5361$

17 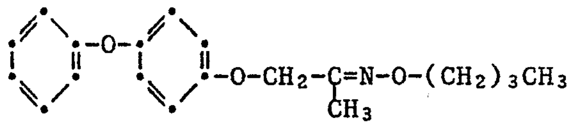 $n_D^{17} = 1,5436$

18 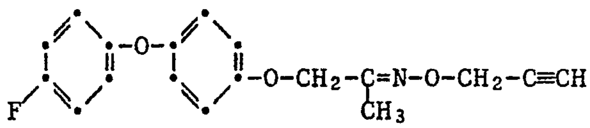 $n_D^{20} = 1,5640$

19 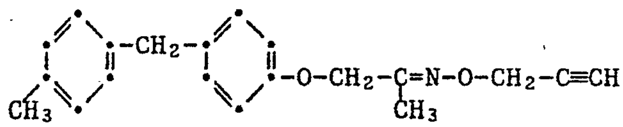 $n_D^{20} = 1,5470$

20 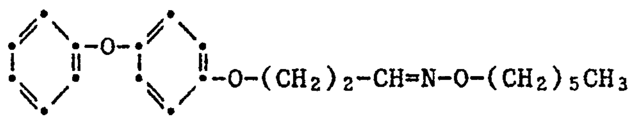 $n_D^{20} = 1,5465$

21 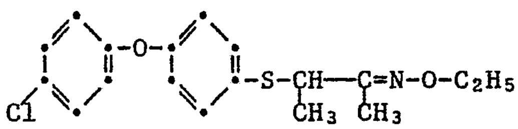 $n_D^{20} = 1,5385$

Beispiel 7:

Formulierungen für flüssige Wirkstoffe der Formel I gemäss Beispiel 1 bis 6 (% = Gewichtsprozent)

| 1. Emulsions-Konzentrate | a) | b) | c) |
|---|---|---|---|
| Wirkstoff gemäss Herstellungsbeispielen | 25 % | 40 % | 50 % |
| Ca-Dodecylbenzolsulfonat | 5 % | 8 % | 6 % |
| Ricinusöl-polyäthylenglykoläther (36 Mol AeO) | 5 % | - | - |

| | | | |
|---|---|---|---|
| Tributylphenol-polyäthylenglykoläther (30 Mol AeO) | - | 12 % | 4 % |
| Cyclohexanon | - | 15 % | 20 % |
| Xylolgemisch | 65 % | 25 % | 20 % |

Aus solchen Konzentraten können durch Verdünnen mit Wasser Emulsionen jeder gewünschten Konzentration hergestellt werden.

| 2. Lösungen | a) | b) | c) | d) |
|---|---|---|---|---|
| Wirkstoff gemäss Herstellungsbeispielen | 80 % | 10 % | 5 % | 95 % |
| Aethylenglykol-monomethyläther | 20 % | - | - | - |
| Polyäthylenglykol (MG 400) | - | 70 % | - | - |
| N-Methyl-2-pyrrolidon | - | 20 % | - | - |
| Epoxidiertes Kokosnussöl | - | - | 1 % | 5 % |
| Benzin (Siedegrenzen 160-190°C) | - | - | 94 % | - |

Die Lösungen sind zur Anwendung in Form kleinster Tropfen geeignet.

| 3. Granulate | a) | b) |
|---|---|---|
| Wirkstoff gemäss Herstellungsbeispielen | 5 % | 10 % |
| Kaolin | 94 % | - |
| Hochdisperse Kieselsäure | 1 % | - |
| Attapulgit | - | 90 % |

Der Wirkstoff wird in Methylenchlorid gelöst, auf den Träger aufgesprüht und das Lösungsmittel anschliessend im Vakuum abgedampft.

| 4. Stäubemittel | a) | b) |
|---|---|---|
| Wirkstoff gemäss Herstellungsbeispielen | 2 % | 5 % |
| Hochdisperse Kieselsäure | 1 % | 5 % |
| Talkum | 97 % | - |
| Kaolin | - | 90 % |

Durch inniges Vermischen der Trägerstoffe mit dem Wirkstoff erhält man gebrauchsfertige Stäubemittel.

Formulierungen für feste Wirkstoffe der Formel I gemäss Beispiel 1 bis 6 (% = Gewichtsprozent)

| 5. Spritzpulver | a) | b) | c) |
|---|---|---|---|
| Wirkstoff gemäss Herstellungsbeispielen | 20 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyäthylenglykoläther (7-8 Mol AeO) | - | 2 % | - |
| Hochdisperse Kieselsäure | 5 % | 10 % | 10 % |
| Kaolin | 67 % | 27 % | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| 6. Emulsions-Konzentrat | a) | b) |
|---|---|---|
| Wirkstoff gemäss Herstellungsbeispielen | 10 % | 10 % |
| Octylphenolpolyäthylenglykoläther (4-5 Mol AeO) | 3 % | - |
| Ca-Dodecylbenzolsulfonat | 3 % | - |
| Ricinusölpolyglykoläther (36 Mol AeO) | 4 % | - |
| Ricinusölthioxilat | - | 25 % |
| Cyclohexanon | 30 % | - |
| Butanol | - | 15 % |

| | | |
|---|---|---|
| Xylolgemisch | 50 % | - |
| Essigester | - | 50 % |

Aus diesem Konzentrat können durch Verdünnen mit Wasser Emulsionen jeder gewünschten Konzentration hergestellt werden.

| 7. Stäubemittel | a) | b) |
|---|---|---|
| Wirkstoff gemäss Herstellungsbeispielen | 5 % | 8 % |
| Talkum | 95 % | - |
| Kaolin | - | 92 % |

Man erhält anwendungsfertige Stäubemittel, indem der Wirkstoff mit dem Träger vermischt und auf einer geeigneten Mühle vermahlen wird.

| 8. Extruder Granulat | |
|---|---|
| Wirkstoff gemäss Herstellungsbeispielen | 10 % |
| Na-Ligninsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert und anschliessend im Luftstrom getrocknet.

| 9. Umhüllungs-Granulat | |
|---|---|
| Wirkstoff gemäss Herstellungsbeispielen | 3 % |
| Polyäthylenglykol (MG 200) | 3 % |
| Kaolin | 94 % |

Der feingemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

10. Suspensions-Konzentrat

| | |
|---|---|
| Wirkstoff gemäss Herstellungsbeispielen | 40 % |
| Aethylenglykol | 10 % |
| Nonylphenolpolyäthylenglykoläther (15 Mol AeO) | 6 % |
| Na-Ligninsulfonat | 10 % |
| Carboxymethylcellulose | 1 % |
| 37%ige wässrige Formaldehyd-Lösung | 0,2 % |
| Silikonöl in Form einer 75%igen wässrigen Emulsion | 0,8 % |
| Wasser | 32 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

Beispiel 8: Insektizide Frassgift-Wirkung: Spodoptera littoralis

Baumwollpflanzen werden mit einer Versuchslösung, enthaltend 400 ppm der zu prüfenden Verbindung, besprüht.

Nach dem Antrocknen des Belages werden die Pflanzen mit Larven von Spodoptera littoralis ($L_1$-Stadium) besetzt. Man verwendet pro Versuchsverbindung zwei Pflanzen. Die Auswertung der erzielten Abtötungsrate erfolgt nach 2, 4, 24, 48 und 72 Stunden. Der Versuch wird bei 28°C und 60 % relativer Luftfeuchtigkeit durchgeführt. Verbindungen gemäss der Formel I gemäss den Beispielen 1 bis 6 zeigen im obigen Test gute Wirkung gegenüber Spodoptera littoralis-Larven.

Beispiel 9: Wirkung gegen Lucilia sericata

Zu 9 ml eines Zuchtmediums wird bei 50°C ein ml einer 0,1 % Aktivsubstanz enthaltenden wässrigen Zubereitung hinzugefügt. Nun werden ca. 30 frisch geschlüpfte Lucilia sericata-Larven zum Zuchtmedium gegeben. Nach 48 und 96 Stunden wird die insektizide Wirkung durch

Ermittlung der Abtötungsrate festgestellt. Verbindungen der Formel I gemäss den Beispielen 1 bis 6 zeigen in diesem Test gute Wirkung gegen Lucilia sericata.

Beispiel 10: Wirkung gegen Aëdes aegypti

Auf die Oberfläche von 150 ml Wasser, das sich in einem Behälter befindet, wird so viel einer 0,1%igen acetonischen Lösung des Wirkstoffs pipettiert, dass eine Konzentration von 100 ppm erhalten wird. Nach Verdunsten des Acetons wird der Behälter mit 30 bis 40 2tägigen Aëdes-Larven beschickt. Nach 2 und 7 Tagen wird die % Mortalität (Anzahl der schwimmunfähigen Larven) geprüft.

Verbindungen der Formel I gemäss den Beispielen 1 bis 6 zeigen gute Wirkung (Mortalität) im obigen Test.

Beispiel 11: Ovizide Wirkung auf Heliothis virescens

Entsprechende Mengenanteile einer benetzbaren pulverförmigen Formulierung, enthaltend 25 Gew.% des zu prüfenden Wirkstoffes, werden mit jeweils so viel Wasser vermischt, dass sich wässrige Emulsionen mit Wirkstoffkonzentrationen von 400 und 200 ppm ergeben.

In diese wirkstoffhaltigen Emulsionen werden eintägige Eigelege von Heliothis auf Cellophan® während drei Minuten eingetaucht und dann auf Rundfiltern abgenutscht. Die so behandelten Gelege werden in Petrischalen ausgelegt und in der Dunkelheit aufbewahrt. Nach 6 bis 8 Tagen wird die Schlupfrate im Vergleich zu unbehandelten Kontrollen festgelegt. Zur Auswertung wird die zur 100%igen Abtötung der Eier erforderliche Wirkstoffkonzentration bestimmt.

Die erfindungsgemässen Verbindungen Nr. 1 und 4 zeigen bei 200 bzw. 400 ppm 100%ige Wirkung (Mortalität) in obigem Test.

Beispiel 12: Insektizide Kontaktwirkung: Myzus persicae

Etwa 4 cm hohe, in Wasser angezogene Erbsenkeimlinge werden vor Versuchsbeginn je mit ca. 200 Individuen der Spezies Myzus persicae besiedelt. Die so behandelten Pflanzen werden 24 Stunden später mit einer wässrigen Suspension enthaltend 400 ppm der zu prüfenden Verbindung bis zur Tropfnässe besprüht. Man verwendet pro Konzentration zwei Pflanzen. Eine Auswertung der erzielten Abtötungsrate erfolgt 48 Stunden nach Applikation. Der Versuch wird bei 20-22°C und 60 % relativer Luftfeuchtigkeit durchgeführt.

Verbindungen der Formel I gemäss den Beispielen 1 bis 6 zeigen in diesem Test eine gute Wirkung.

Beispiel 13: Wirkung gegen Bodeninsekten (Diabrotica balteata)

Es werden 5 etwa einen bis drei cm lange Maiskeimlinge sowie eine Rondelle aus Filterpapier in eine 400 ppm des zu prüfenden Wirkstoffes enthaltende wässrige Zubereitung getaucht. Die feuchte Filterpapierrondelle wird auf dem Boden eines 200 ml Plastikbechers ausgelegt, und dann werden die 5 behandelten Maiskeimlinge zusammen mit 10 Larven von Diabrotica balteata des zweiten bis dritten Larvalstadiums in den Becher gegeben. Pro Wirkstoffkonzentration werden 2 Ansätze durchgeführt. Die mit den Larven besetzten Becher werden während 6 Tagen bei Tageslicht, einer relativen Luftfeuchtigkeit von 40 bis 60 % und einer Temperatur von 22 bis 24°C gehalten. Danach wird die prozentuale Abtötung der Testtiere bestimmt.

Verbindungen gemäss den Beispielen 1 bis 6 zeigen gute Wirkung in diesem Test.

Beispiel 14: Wirkung auf Laspeyresia pomonella (Eier)

Abgelegte Eier von Laspeyresia pomonella, die nicht älter als 24 Stunden sind, werden auf Filterpapier für 1 Minute in eine acetonisch-wässrige Lösung enthaltend 400 ppm des zu prüfenden Wirkstoffes eingetaucht. Nach dem Antrocknen der Lösung werden die

Eier in Petrischalen ausgelegt und bei einer Temperatur von 28°C belassen. Nach 6 Tagen wird der prozentuale Schlupf aus den behandelten Eiern bewertet.

Die erfindungsgemässen Verbindungen Nr. 2, 3 und 4 zeigen 100%ige Wirkung in obigem Test.

Beispiel 15: Wirkung gegen Spodoptera littoralis (Frasshemmung, Repellentwirkung)

Es werden aus Baumwollblättern Blattrondellen von 4 cm Durchmesser hergestellt, von denen ein Teil durch Eintauchen in eine wässrige Zubereitung des zu prüfenden Wirkstoffes und nachfolgendes Trocknen behandelt wird. Ein rechteckiger flacher Behälter (10 x 20 cm) wird mit 50 Spodoptera-Larven im 2. Larvalstadium besiedelt. Der Behälter ist mit einem Plastik-Deckel abgeschlossen, in dem sich zwei runde Aussparungen (ca. 7 cm Durchmesser) befinden, welche jeweils so durch einen Plastikbecher abgeschlossen sind, dass die Oeffnung des Bechers mit dem Rand der Aussparung abschliesst. Auf dem Boden eines jeden Bechers befindet sich eine Agar-Schicht mit einer darauf haftenden Baumwollblatt-Rondelle, wobei der eine Becher eine behandelte und der andere eine unbehandelte Rondelle enthält.

Nach 18 Stunden bei Raumtemperatur und Neon-Beleuchtung wird auf Frasshemmung (fehlender Frass an der behandelten Rondelle) und Repellentwirkung (keine Larven an der behandelten Rondelle) unter Berücksichtigung des phototaktischen Verhaltens der Test-Larven bonitiert.

Die erfindungsgemässe Verbindung Nr. 10 zeigt bei 200 ppm 100 % Frasshemmung und 100 % Repellentwirkung in diesem Test.

Beispiel 16: Wirkung gegen Zecken: Eiablagehemmung

Als Testtiere werden vollgesogene Weibchen der Rinderzecke Boophilus microplus verwendet. Pro Konzentration werden je 10 Zecken eines OP-resistenten Stammes (z.B. Biarra-Stamm) und eines normal empfindlichen Stammes (z.B. Yeerongpilly-Stamm) behandelt. Die

Zecken werden auf mit Doppelklebeband bezogenen Platten fixiert und dann entweder mit wässrigen Emulsionen bzw. mit Lösungen der Salze der zu untersuchenden Verbindungen benetzt oder mit einem mit diesen Flüssigkeiten getränkten Wattebausch in Berührung gebracht. Anschliessend werden sie in einem klimatisierten Raum bei konstanten Bedingungen aufbewahrt. Die Auswertung erfolgt nach drei Wochen. Es wird die totale Hemmung der Ablage von fertilen Eiern ermittelt.

Die Hemmwirkung der Substanzen wird ausgedrückt als minimale Substanzkonzentration in ppm bei 100%iger Wirkung gegen normal sensible und resistente adulte weibliche Zecken.

In diesem Test zeigen eine 100%ige akarizide Wirkung die Verbindungen Nr. 2, 4 und 10 bei 250 ppm und die Verbindung Nr. 1, 11, 13 und 14 bei 500 ppm.

Patentansprüche

1. Verbindung der Formel I

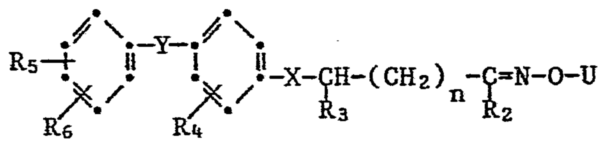 (I),

worin

U für einen der Reste

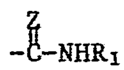 , 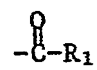 oder $R_1$

steht und wobei

$R_1$ $C_1$-$C_6$-Alkyl, $C_2$-$C_5$-Alkenyl, $C_3$-Monohalogenalkenyl, $C_3$-$C_5$-Alkinyl, $C_3$-$C_6$-Cycloalkyl, $C_2$-$C_5$-Alkoxyalkyl, $C_2$-$C_5$-Alkylthioalkyl oder gegebenenfalls am Kern substituiertes Benzyl;

$R_2$ und $R_3$ unabhängig voneinander Wasserstoff oder $C_1$-$C_3$-Alkyl,

$R_4$, $R_5$ und $R_6$ unabhängig voneinander Wasserstoff, Halogen, $C_1$-$C_4$-Alkyl, $C_2$-$C_4$-Halogenalkyl, $C_1$-$C_4$-Alkoxy, $C_1$-$C_4$-Halogenalkoxy oder Nitro,

n eine Zahl 0 oder 1,

X Sauerstoff, Schwefel oder $>CH_2$,

Y Sauerstoff, Schwefel, $>CH_2$ oder $>C{=}O$ und

Z Sauerstoff oder Schwefel bedeuten; mit der Massgabe, dass X und Y nicht gleichzeitig Sauerstoff bedeuten und/oder mindestens einer der Reste $R_4$, $R_5$ und $R_6$ eine von Wasserstoff abweichende Bedeutung hat, wenn $R_1$ eine $C_1$-$C_3$-Alkylgruppe darstellt.

2. Eine Verbindung der Formel I gemäss Anspruch 1, dadurch gekennzeichnet, dass

$R_1$ $C_1$-$C_6$-Alkyl, $C_2$-$C_5$-Alkenyl, $C_3$-Chloralkenyl, $C_3$-$C_5$-Alkinyl oder gegebenenfalls am Kern substituiertes Benzyl;

$R_2$ und $R_3$ unabhängig voneinander Wasserstoff oder Methyl;

$R_4$, $R_5$ und $R_6$ Wasserstoff,

n eine Zahl 0 oder 1;

X Sauerstoff, Schwefel oder $>CH_2$;
Y Sauerstoff oder $>CH_2$ und

Z Sauerstoff oder Schwefel bedeuten.

3. Verbindung der Formel I gemäss Anspruch 1, dadurch gekennzeichnet, dass
U für einen der Reste

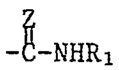 , 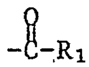 , $-CH_2-CH=CHCl$ ,

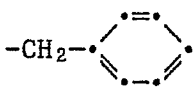 , 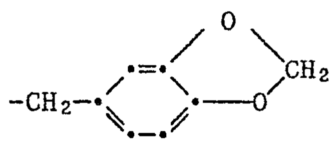 , $-CH_2-C\equiv CH$

oder $-CH_2-CH=CH_2$ steht
und $R_1$, $R_2$, $R_3$, $R_4$, $R_5$, $R_6$, n, X, Y und Z die in Anspruch 1 oder 2 angegebenen Bedeutungen haben, mit der Massgabe, dass Y und X gleichzeitig Sauerstoff, sowie $R_2$ Methyl bedeuten und/oder, dass mindestens einer der Reste $R_4$, $R_5$ oder $R_6$ eine von Wasserstoff abweichende Bedeutung hat, wenn U für den Rest $-CH_2-CH=CH_2$ steht.

4. Verbindung der Formel Ia gemäss Anspruch 3

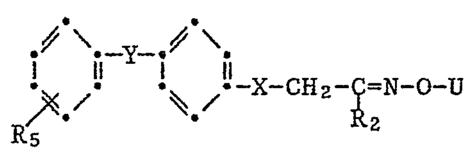 (Ia),

dadurch gekennzeichnet, dass
U für einen der Reste

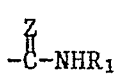 , $-CH_2-CH=CHCl$ , 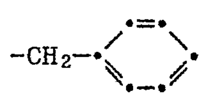 ,

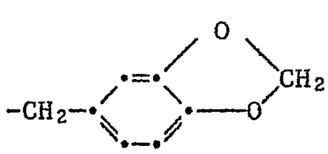 oder $-CH_2-CH=CH_2$

steht und

$R_1$ $C_1$-$C_4$-Alkyl;

$R_2$ Wasserstoff oder Methyl;

$R_5$ Wasserstoff, Halogen, Methyl oder Trifluormethyl;

X und Z unabhängig voneinander Sauerstoff oder Schwefel; und

Y Sauerstoff oder $>CH_2$ bedeuten, mit der Massgabe, dass X und Y gleichzeitig Sauerstoff und $R_2$ Methyl bedeuten und/oder, dass $R_3$ eine von Wasserstof abweichende Bedeutung hat, wenn U für den Rest $-CH_2-CH=CH_2$ steht.

5. Verbindung gemäss Anspruch 4 der Formel

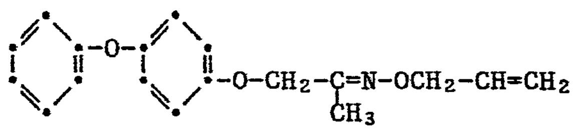 .

6. Verbindung gemäss Anspruch 2 der Formel

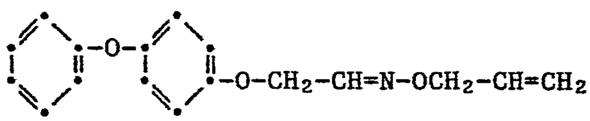 .

7. Verbindung gemäss Anspruch 2 der Formel

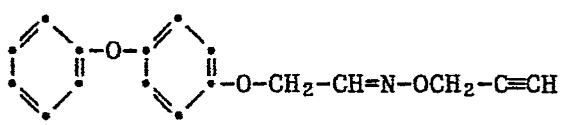 .

8. Verbindung gemäss Anspruch 3 der Formel

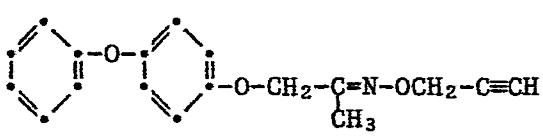 .

9. Verbindung gemäss Anspruch 1 der Formel

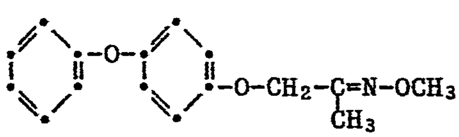 .

10. Verbindung gemäss Anspruch 1 der Formel

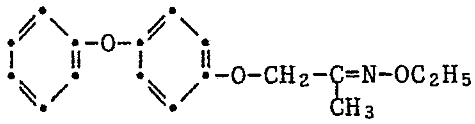
.

11. Verbindung gemäss Anspruch 3 der Formel

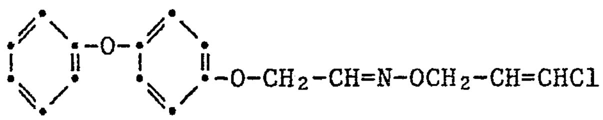
.

12. Verbindung gemäss Anspruch 3 der Formel

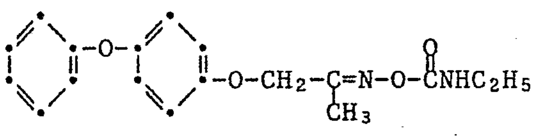
.

13. Verbindung gemäss Anspruch 3 der Formel

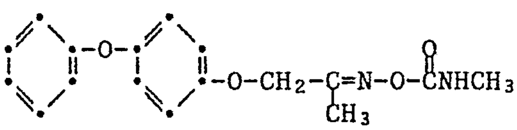
.

14. Verbindung gemäss Anspruch 3 der Formel

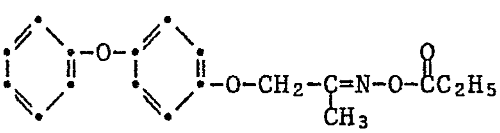
.

15. Verfahren zur Herstellung einer Verbindung gemäss einem der Ansprüche 1 bis 14, dadurch gekennzeichnet, dass man eine Verbindung der Formel III

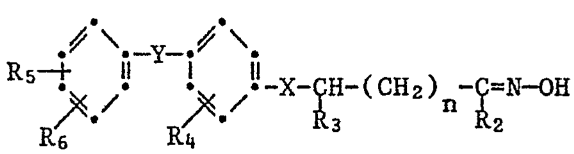
(III)

a) mit einer Verbindung der Formel $Z=C=N-R_1$ (IV) oder

b) mit einer Verbindung der Formel $Hal-CO-R_1$ (V) oder mit einer Verbindung der Formel $O(CO-R_1)_2$ (VI) oder

c) mit einer Verbindung der Formel $A-R_1$ umsetzt, wobei die Reste $R_1$-$R_6$, n, X, Y und Z die in den Ansprüchen 1 bis 4 angegebenen Bedeutungen haben, Hal für ein Halogenatom und A für eine Abgangsgruppe steht.

16. Verfahren zur Herstellung einer Verbindung der Formel I gemäss einem der Ansprüche 1 bis 11, worin U die Bedeutung $R_1$ hat, dadurch gekennzeichnet, dass man eine Verbindung der Formel II

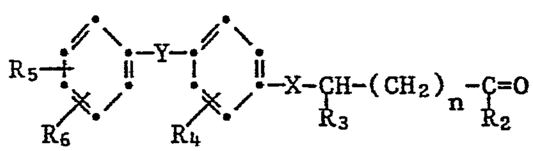

(II)

mit einer Verbindung der Formel VIII

$$\left[ H_3\overset{\oplus}{N}-O-R_1 \right] \quad Hal^{\ominus} \qquad \text{(VIII)}$$

in Gegenwart einer Base umsetzt, wobei die Reste $R_1$ bis $R_6$, n, X und Y die in den Ansprüchen 1 bis 4 angegebenen Bedeutungen haben und Hal für ein Halogenatom steht.

17. Schädlingsbekämpfungsmittel, welches als aktive Komponente eine Verbindung gemäss einem der Ansprüche 1 bis 14 und in der Formulierungstechnik übliche Hilfsmittel enthält.

18. Verwendung einer Verbindung gemäss einem der Ansprüche 1 bis 14 zur Bekämpfung von Schädlingen an Tieren und Pflanzen.

19. Verwendung gemäss Anspruch 18 zur Bekämpfung von Insekten und Vertretern der Ordnung Akarina.

20. Verwendung gemäss Anspruch 19 als Ovizid zur Bekämpfung pflanzenschädigender Insekten.

21. Verfahren zur Bekämpfung von Schädlingen an Tieren und Pflanzen, dadurch gekennzeichnet, dass man eine Verbindung gemäss einem der Ansprüche 1 bis 14 auf die Tiere, Pflanzen oder deren Aufenthaltsort oder Standort appliziert.

FO 7.5 EIC/bg*